# EUROPEAN PATENT APPLICATION

(11) **EP 4 481 061 A1**
(43) Date of publication of application: **25.12.2024**
(21) Application number: 23756619.5
(22) Date of filing: 15.02.2023
(51) Int. Cl.: C12Q 1/6883

(54) **LONG NON-CODING RNA BIOMARKER IN EXOSOMES FOR DIAGNOSING ATRIAL FIBRILLATION AND USE THEREOF**

(30) Priority: 15.02.2022 KR 20220019791
(71) Applicant: Yonsei University, University-Industry Foundation(UIF)., Seoul 03722 (KR)
(72) Inventor: JOUNG, Boyoung, Seoul 06713 (KR); KANG, Ji-Young, Seoul 03722 (KR); YUN, Nuri, Seoul 03722 (KR); MUN, Dasom, Seoul 03722 (KR); KIM, Hyoeun, Seoul 03722 (KR); CHUN, Yumin, Seoul 03722 (KR)
(74) Representative: AWA Sweden AB
(86) International application number: PCT/KR2023/002195
(87) International publication number: WO 2023/158207

(57) **Abstract**

The present invention provides a long non-coding RNA (lncRNA) biomarker composition in serum exosomes, comprising LOC105377989, LOC105375434, LOC107986997, and LOC101927073, for diagnosing atrial fibrillation on the basis of the results of profile analysis of lncRNA in serum exosomes of patients with atrial fibrillation. When used, the lncRNA biomarker composition enables non-invasive *in-vitro* diagnosis, and exhibits an excellent effect in atrial fibrillation diagnosis, and thus is highly applicable as a useful diagnostic biomarker for atrial fibrillation.

## Description

### [Technical Field]

This application claims priority to and the benefit of Korean Patent Application No. 10-2022-0019791, filed on February 15, 2022, the disclosure of which is incorporated herein by reference in its entirety.

The present invention relates to a lncRNA biomarker which is capable of diagnosing atrial fibrillation.

The present invention was completed by Project Number 2021M3ESE5096196 (21B0604L1-01, 1711149581) with the support of the Ministry of Health and Welfare of the Republic of Korea and Project Number 2021R1I1A1A01052197 (1345333178) with the support of the Ministry of Education of the Republic of Korea.

### [Background Art]

Atrial fibrillation (AF) is a type of arrhythmia (irregular pulse) in which the heart beats irregularly, and it is a representative heart disease in which the number of patients is rapidly increasing worldwide in line with the aging society. Atrial fibrillation is often asymptomatic, and it greatly increases the risk of severe diseases such as death, stroke, heart failure, vascular dementia and the like. Although atrial fibrillation is not a rare disease, there is no fundamental diagnosis and treatment method so far, and thus, the unmet medical demand is very large. Because of this importance, it is important to diagnose atrial fibrillation early and with high accuracy.

Meanwhile, exosomes, which are biological nanoparticles that have a size of 30 to 150 nm and are secreted from various cells, are known to circulate in body fluids by including biomaterials (mRNA/non-coding RNA and proteins, etc.), and recently, it has been reported that biomaterials in exosomes can serve as important markers reflecting the current disease stage or health status.

Korean Patent Application Laid-Open No. 10-2015-0054558 discloses that lipoproteins in the serum of patients can be used for the diagnosis of atrial fibrillation by utilizing the same as biomarkers, but there is no disclosure about using lncRNAs as biomarkers.

Therefore, since the expression patterns of lncRNAs in exosomes are different in various diseases, it is necessary to select atrial fibrillation-specific lncRNA biomarkers in exosomes, which are being actively studied in the medical field as non-invasive diagnostic biomarkers, for the personalized diagnosis of atrial fibrillation.

Accordingly, the inventors of the present invention completed the present invention by isolating exosomes from samples and identifying lncRNA biomarkers for the diagnosis and prediction of atrial fibrillation through two validation phases.

### [Disclosure]

### [Technical Problem]

Accordingly, the inventors of the present invention have made diligent efforts to discover a non-invasive and accurate diagnostic marker for atrial fibrillation, and as a result, the present invention was completed by identifying a new biomarker which is capable of predicting the presence or absence of atrial fibrillation through RNA sequencing.

Therefore, an object of the present invention is to provide a biomarker composition for diagnosing atrial fibrillation, including at least one lncRNA selected from the group consisting of LOC105377989, LOC105375434, LOC107986997 and LOC101927073 as an active ingredient.

In addition, another object of the present invention is to provide a biomarker composition for diagnosing atrial fibrillation, including an agent for measuring the expression level of at least one lncRNA selected from the group consisting of LOC105377989, LOC105375434, LOC107986997 and LOC101927073.

Still another object of the present invention is to provide a kit for diagnosing atrial fibrillation, including the composition.

In addition, another object of the present invention is to provide a method for providing information that is necessary for diagnosing atrial fibrillation, including the step of measuring the expression level of at least one lncRNA selected from the group consisting of LOC105377989, LOC105375434, LOC107986997 and LOC101927073 in a sample isolated from a subject.

Still another object of the present invention is to provide a method for screening a therapeutic agent for atrial fibrillation, including the step of treating an atrial fibrillation therapeutic agent candidate material to exosomes isolated from mammals with atrial fibrillation except humans or atrial fibrillation patients, and then measuring the expression level of at least one lncRNA selected from the group consisting of LOC105377989, LOC105375434, LOC107986997 and LOC101927073.

### [Technical Solution]

The terms used in the present specification are used for descriptive purposes only and should not be construed as restrictive. Singular expressions include plural expressions unless the context clearly dictates otherwise. In the present specification, terms such as "include" or "have" are intended to designate that there is a feature, number, step, operation, component, part or combination thereof described in the specification, but it should be understood that this does not preclude the presence or additional possibility of one or more other features, numbers, steps, operations, components, parts or combinations thereof in advance. Unless defined otherwise, all terms used herein, including technical or scientific terms, have the same meaning as commonly understood by a person of ordinary skill in the art to which the exemplary embodiments pertain. Terms such as those defined in commonly used dictionaries should be interpreted as having a meaning consistent with the meaning in the context of the related art, and unless explicitly defined in the present application, they should not be interpreted in an ideal or excessively formal meaning.

In order to achieve the above objects, the present invention provides a biomarker composition for diagnosing atrial fibrillation, including at least one lncRNA selected from the group consisting of LOC105377989, LOC105375434, LOC107986997 and LOC101927073 as an active ingredient.

In addition, the present invention provides a biomarker composition for diagnosing atrial fibrillation, including an agent for measuring the expression level of at least one lncRNA selected from the group consisting of LOC105377989, LOC105375434, LOC107986997 and LOC101927073.

In an exemplary embodiment of the present invention, the agent for measuring the expression level of the lncRNA may be a primer or probe that specifically binds to the lncRNA.

The present invention provides a kit for diagnosing atrial fibrillation, including the composition.

The present invention provides a method for providing information that is required for diagnosing atrial fibrillation, including the step of measuring the expression level of at least one lncRNA selected from the group consisting of LOC105377989, LOC105375434, LOC107986997 and LOC101927073 in a sample isolated from a subject.

In an exemplary embodiment of the present invention, the sample may be blood or blood-derived serum exosomes.

In an exemplary embodiment of the present invention, the method may further include the step of determining atrial fibrillation when the result of comparing the measured expression level of lncRNA with the expression level of lncRNA of a normal control sample corresponds to any one of a) to d) below:
a) upregulation of LOC105377989;
b) upregulation of LOC105375434;
c) upregulation of LOC107986997; and
d) downregulation of LOC101927073.

In addition, the present invention provides a method for screening a therapeutic agent for atrial fibrillation, including the step of treating an atrial fibrillation therapeutic agent candidate material to exosomes isolated from mammals with atrial fibrillation except humans or atrial fibrillation patients, and then measuring the expression level of at least one lncRNA selected from the group consisting of LOC105377989, LOC105375434, LOC107986997 and LOC101927073.

In an exemplary embodiment of the present invention, the method may further include the step of determining an atrial fibrillation therapeutic agent, when the expression level of lncRNA after treatment with the atrial fibrillation therapeutic agent candidate material compared to before treatment with the atrial fibrillation therapeutic agent candidate material corresponds to any one of a) to d) below:
a) downregulation of LOC105377989;
b) downregulation of LOC105375434;
c) downregulation of LOC107986997; and
d) upregulation of LOC101927073.

As described above, it has become important to diagnose atrial fibrillation with high accuracy at the early stage, and since biomaterials in exosomes, which are actively studied in the medical world as non-invasive diagnostic biomarkers, have different expression patterns in various diseases, it was required to screen lncRNA biomarkers in atrial fibrillation-specific exosomes. The inventors of the present invention completed the present invention by isolating exosomes from samples to discover non-invasive and accurate atrial fibrillation diagnostic markers, and identifying lncRNA biomarkers for the diagnosis and prediction of atrial fibrillation through two validation phases.

Accordingly, the present invention provides a biomarker composition for diagnosing atrial fibrillation, including at least one lncRNA selected from the group consisting of LOC105377989, LOC105375434, LOC107986997 and LOC101927073 as an active ingredient.

As used herein, the term "biomarker" refers to a molecule that is quantitatively or qualitatively associated with the presence of a biological phenomenon, and the biomarker of the present invention may be a lncRNA that can confirm the presence of atrial fibrillation. The term includes nucleic acid sequences that are complementary to or flanking a marker sequence, such as nucleic acids used as probes or primer pairs that are capable of amplifying the marker sequence.

The "lncRNA (long non-coding RNA)" is a recently discovered new class of transcript that is infiltratively transcribed into the genome, and it is an important regulator of the epigenome. The lncRNA may be a useful biomarker for predicting and diagnosing poor prognosis in some heart diseases.

The lncRNA has recently been re-examined as a regulatory RNA, and particularly plays a major role in epigenetics, or affects transcriptional regulation, cancer formation, embryonic development, nervous system disorders or other essential biological processes.

According to an exemplary embodiment of the present invention, after extracting and comparing RNA from exosomes in the serum of non-atrial fibrillation and atrial fibrillation groups, the biomarkers that are differentially expressed between the two groups are identified, and thus, it was confirmed that the biomarkers can be utilized as diagnostic markers for atrial fibrillation.

In the present invention, "diagnosis" means confirming the presence or character of a pathological condition. Diagnosis in the present invention is to determine the presence or occurrence of atrial fibrillation pathology by measuring the expression level of at least one lncRNA selected from the group consisting of LOC105377989, LOC105375434, LOC107986997 and LOC101927073.

According to another aspect of the present invention, provided is a biomarker composition for diagnosing atrial fibrillation, including an agent for measuring the expression level of at least one lncRNA selected from the group consisting of LOC105377989, LOC105375434, LOC107986997 and LOC101927073.

The "agent" or "test agent" may include any substance, molecule, element, compound, entity or combination thereof. For example, it may include proteins, polypeptides, small organic molecules, polysaccharides, polynucleotides and the like, and may be a natural product, synthetic compound or chemical compound, or a combination of two or more substances. Unless defined otherwise, the agent, substance and compound may be used interchangeably.

In addition, the present invention provides a composition for diagnosing atrial fibrillation, including a substance for measuring the expression level of the lncRNAs of the two types of biomarkers of the present invention.

In an exemplary embodiment of the present invention, the agent for measuring the expression level of the lncRNA may be a primer or probe that specifically binds to the lncRNA.

In the present invention, a "primer" is a nucleic acid sequence having a short free 3' hydroxyl group, and refers to a short nucleic acid sequence which can form a base pair with a complementary template and function as a starting point for copying the template. In the present invention, PCR amplification is performed by using sense and antisense primers of the 4 types of biomarker lncRNAs described above, and atrial fibrillation can be diagnosed through whether desired products are produced. The PCR conditions and lengths of sense and antisense primers may be modified based on those known in the art.

As used herein, the term "probe" refers to a nucleic acid fragment such as RNA or DNA corresponding to as short as several bases to as long as several hundred bases that are capable of specific binding to lncRNA, and since it is is labeled, the presence or absence of specific lncRNAs can be confirmed. The probe may be fabricated in the form of an oligonucleotide probe, a single stranded DNA probe, a double stranded DNA probe, an RNA probe and the like. In the present invention, atrial fibrillation may be diagnosed through hybridization, by performing hybridization using the biomarker lncRNA of the present invention and a complementary probe. The selection of suitable probes and hybridization conditions may be modified based on those known in the art.

Primers or probes of the present invention can be chemically synthesized by using the phosphoramidite solid support method or other well-known methods. Such nucleic acid sequences can also be modified by using a number of means known in the art. Non-limiting examples of such modifications include methylation, capping, substitution of one or more homologs of the natural nucleotide, and modifications between nucleotides, such as uncharged linkages (e.g., methyl phosphonates, phosphotriesters, phosphoro amidates, carbamates, etc.) or to charged linkages (e.g., phosphorothioates, phosphorodithioates, etc.).

The present invention provides a kit for diagnosing atrial fibrillation, including the composition.

According to a preferred exemplary embodiment of the present invention, the kit may be a RT-PCR kit, a competitive RT-PCR kit, a real-time RT-PCR kit, a DNA chip kit or a protein chip kit.

The kit of the present invention may include a composition, solution or device including primers and probes that recognize the 4 types of biomarker lncRNAs, as well as one or more other components that are suitable for an analysis method.

In addition, the present invention provides a method for providing information that is required for diagnosing atrial fibrillation, including the step of measuring the expression level of at least one lncRNA selected from the group consisting of LOC105377989, LOC105375434, LOC107986997 and LOC101927073 in a sample isolated from a subject.

In the present invention, a "subject" is an individual at risk of heart disease or atrial fibrillation, and it may include mammals such as humans, primates including chimpanzees, pets such as dogs and cats, livestock animals such as cows, horses, sheep and goats, rodents such as mice and rats, farmed fish and the like without limitation.

In the present invention, a "sample" used for analysis includes biological samples that can identify atrial fibrillation-specific lncRNA that can be distinguished from normal conditions, such as blood, plasma, serum, saliva, nasal fluid, sputum, ascites, vaginal secretions, urine, feces and the like. Preferably, it may be a biological liquid sample, such as blood, serum, plasma or urine, and most preferably, blood or blood-derived serum exosomes. The sample may be prepared to increase the detection sensitivity of lncRNA markers, and for example, samples obtained from patients may be pre-treated by using methods such as anion exchange chromatography, affinity chromatography, size exclusion chromatography, liquid chromatography or sequential extraction.

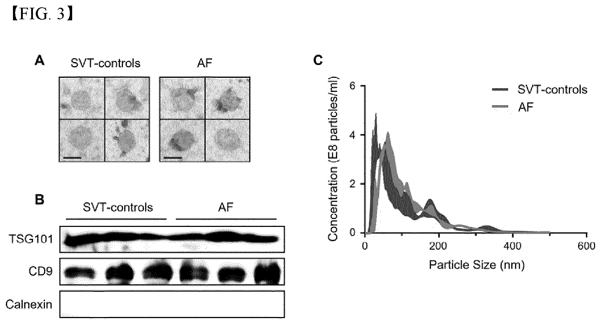

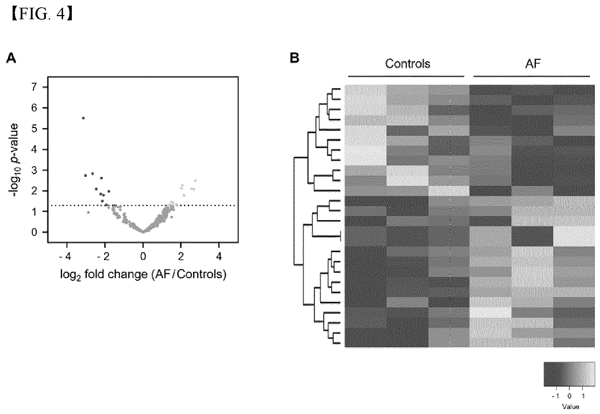

In an exemplary embodiment of the present invention, the sample may be blood or blood-derived serum exosomes. Specifically, in an exemplary embodiment of the present invention, serum was separated by centrifuging the blood of a patient diagnosed with atrial fibrillation at room temperature (about 25°C at 3,000 rpm for 7 minutes). Exosomes were isolated from the separated serum, and the characteristics of exosomes were identified by using transmission electron microscopy, nanoparticle tracking analysis and Western blotting techniques. It was confirmed that exosomes were successfully isolated from serum through the detection of typical exosome shape (circular shape), size and protein markers (FIGS. 3A to C). After extracting RNA from exosomes, RNA-sequencing was performed to preferentially identify lncRNAs that were differentially expressed (FIG. 4), and after two validation processes, the atrial fibrillation biomarker was finally selected. When blood or serum exosomes are used as biomarkers, non-invasive and *in vitro* diagnosis is possible.

In an exemplary embodiment of the present invention, the method may further include the step of determining atrial fibrillation when the result of comparing the measured expression level of lncRNA with the expression level of lncRNA of a normal control sample corresponds to any one of a) to d) below:
a) upregulation of LOC105377989;
b) upregulation of LOC105375434;
c) upregulation of LOC107986997; and
d) downregulation of LOC101927073.

The normal control group refers to a non-atrial fibrillation group without atrial fibrillation, and it may specifically refer to a group with symptoms of premature atrial contraction but no atrial fibrillation. Atrial fibrillation disease may be accurately determined by obtaining and comparing lncRNA expression levels or expression patterns in samples collected from normal controls and patients and samples collected from patients in which the presence or prognosis of atrial fibrillation is to be determined.

In addition, the present invention provides a method for screening a therapeutic agent for atrial fibrillation, including the step of treating an atrial fibrillation therapeutic agent candidate material to exosomes isolated from mammals with atrial fibrillation except humans or atrial fibrillation patients, and then measuring the expression level of at least one lncRNA selected from the group consisting of LOC105377989, LOC105375434, LOC107986997 and LOC101927073.

In an exemplary embodiment of the present invention, the method may further include the step of determining an atrial fibrillation therapeutic agent, when the expression level of lncRNA after treatment with the atrial fibrillation therapeutic agent candidate material compared to before treatment with the atrial fibrillation therapeutic agent candidate material corresponds to any one of a) to d) below:
a) downregulation of LOC105377989;
b) downregulation of LOC105375434;
c) downregulation of LOC107986997; and
d) upregulation of LOC101927073.

Increasing or suppressing the expression of the lncRNA is sufficient as long as it can interact with the lncRNA to affect the activity thereof, and at least one selected from the group consisting of antibodies or antigen-binding fragments thereof, aptamers, siRNA, shRNA, microRNA, inhibitory compounds and pharmaceutically acceptable salts thereof may be used, but the present invention is not limited thereto.

The method for screening a therapeutic agent for atrial fibrillation according to the present invention may further include the step of determining a candidate material as a therapeutic agent for atrial fibrillation, when the expression level of at least one lncRNA selected from the group consisting of LOC105377989, LOC105375434 and LOC107986997 measured above is downregulated compared to before treating the candidate material. Alternatively, the same applies if the measured expression level of LOC101927073 lncRNA is upregulated compared to before treating the candidate material. Specifically, it may be advantageously used to screen a therapeutic agent for atrial fibrillation by comparing an increase or decrease in the lncRNA expression of a marker under the presence or absence of an atrial fibrillation treatment candidate material. That is, the expression levels of the 4 types of biomarker lncRNAs of the present invention are measured in biological samples isolated from patients who have exhibited atrial fibrillation in the absence of a treatment candidate material for atrial fibrillation, and the expression levels of the 4 types of biomarker lncRNAs of the present invention are measured in the presence of a treatment candidate material for atrial fibrillation to compare both cases, and then, the material that increases or decreases the expression levels in the presence of a treatment candidate material compared to the expression level in the absence thereof may be selected as a therapeutic agent for atrial fibrillation.

Since the method for providing information that is required for diagnosing atrial fibrillation and the method for screening a therapeutic agent for atrial fibrillation treatment according to the present invention are all *in vitro* methods that are performed on samples isolated from subjects, they do not target humans or the human body.

However, when the method for screening a therapeutic agent for atrial fibrillation treatment is performed *in vivo,* it targets mammals except humans, and it does not target humans or the human body.

### [Advantageous Effects]

The lncRNA according to the present invention is a biomarker for diagnosing atrial fibrillation, and it can improve patient convenience and effectively diagnose or prevent atrial fibrillation in a minimally invasive manner.

### [Description of Drawings]

FIG. 1 shows a mimetic diagram of the entire process for discovering lncRNAs in atrial fibrillation-specific exosomes. The present invention proceeded through exosome isolation, screening and two independent validation phases.
FIG. 2 shows patient group information used in the present invention.
FIG. 3 shows the results of serum exosome isolation and verification.
FIG. 4 shows the results of sequencing profiles of lncRNAs in serum exosomes.
FIG. 5 shows the first validation of lncRNAs in serum exosomes.
FIG. 6 shows the second validation of lncRNAs in serum exosomes.
FIG. 7 shows the target gene prediction results of lncRNAs in serum exosomes.

### [Modes of the Invention]

### [Example 1]

### Selection of patients and preparation of blood samples

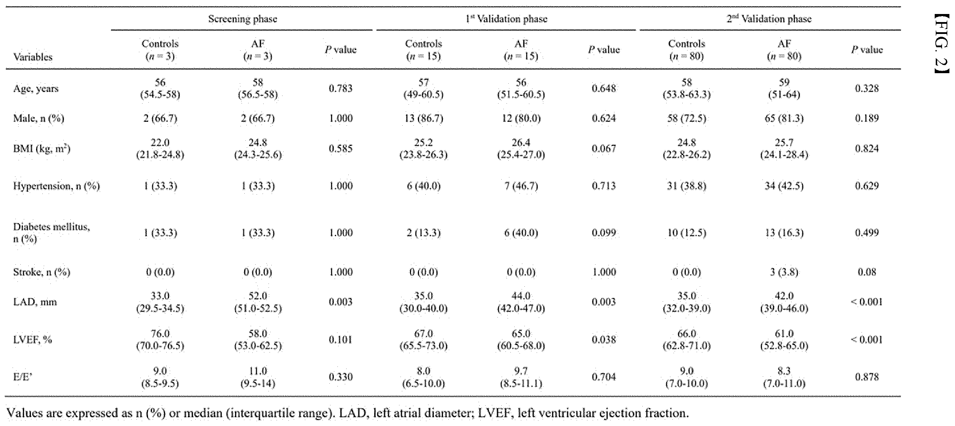

The present invention was targeted for a total of 95 normal controls (non-atrial fibrillation group) and 95 atrial fibrillation patients (atrial fibrillation group) who underwent radiofrequency (RF) catheter ablation at Yonsei Medical Center (Seoul, Korea) from August 2018 to July 2021. The non-atrial fibrillation group, which was a normal control group, was composed of patients with symptoms of premature atrial contraction but no atrial fibrillation. This was approved by the Institutional Review Board (IRB) of Yonsei University Health System Severance Hospital (Approval No. 4-2011-0872) and complied with the principles of the Declaration of Helsinki. Specific patient information is shown in [FIG. 2].

### [Example 2]

### Isolation of serum exosomes and validation

The blood of patients diagnosed with atrial fibrillation was centrifuged at 3,000 rpm for 7 minutes at room temperature (about 25°C) to separate serum. Exosomes were isolated from the separated serum by using the Exoquick^{™} Exosome precipitation kit (System Bioscience (SBI)). The exosomes of the normal control group (non-atrial fibrillation group) without atrial fibrillation were named Controls, and the exosomes of the atrial fibrillation patient group (atrial fibrillation group) were named AF. The isolated exosomes were resuspended in 1X PBS (phosphate buffered saline), and exosome characteristics were characterized by transmission electron microscopy, nanoparticle tracking analysis and Western blotting techniques. Through the detection of typical exosome shape (circular shape), size (average particle size distribution Controls = 108.0 ± 15.6 nm, AF = 114.0 ± 4.2 nm) and protein markers (TSG 101 and CD81), it was confirmed that exosomes were successfully isolated from serum (FIGS. 3A-C).

### [Example 3]

### Sequencing profile of lncRNAs in serum exosomes

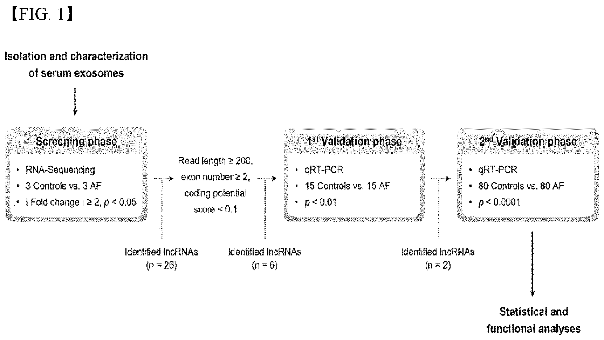

The process of selecting biomarker candidates is summarized in [FIG. 1]. In order to screen biomarker candidates for atrial fibrillation, after RNA was extracted from exosomes in the serum of the non-atrial fibrillation group and the atrial fibrillation group with QIAzol Lysis Reagent (Qiagen), RNA-sequencing was performed. As a result, it was confirmed that a total of 399 lncRNAs were differentially expressed between the two groups (FIG. 4A). Yellow, blue and gray dots in FIG. 3A represent significantly up- and down-regulated exosomes, lncRNAs with p < 0.05 and lncRNAs with p ≥ 0.05, respectively. Among these, 26 lncRNAs (15 upregulated lncRNAs and 11 downregulated lncRNAs) had a |fold change| ≥ 2 (p < 0.05) which showed a distinct expression pattern between the two groups (FIG. 4B).

Afterwards, among the lncRNAs satisfying the conditions of (1) read length ≥ 200 nucleotides, (2) exon number ≥ 2, and (3) coding potential score < 0.1, a total of 6 lncRNAs in exosomes (LOC105377989, LOC105375434, LOC107986997, LOC101927073, LINC01705 and LOC102723403) showing the most significant expression patterns between the two groups were finally screened for the first validation phase.

### [Example 4]

### First validation of lncRNAs in serum exosomes

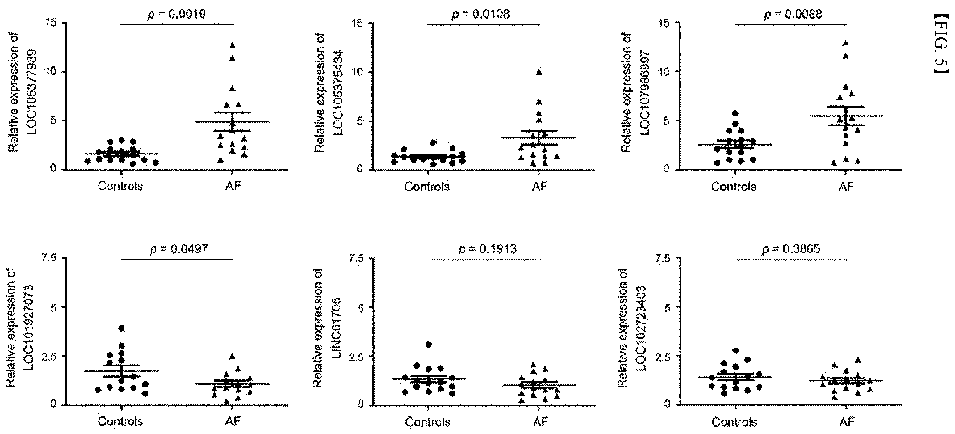

In the first validation phase consisting of 15 patients of the non-atrial fibrillation group and 15 patients of the atrial fibrillation group, qRT-PCR was performed to determine the expression levels of lncRNAs in the 6 candidate exosomes identified in the screening phase. As a result, the expression levels of 4 lncRNAs in exosomes (LOC105377989, LOC105375434, LOC107986997 and LOC101927073) showed significantly differential expressions between the two groups (all, p < 0.05). On the other hand, the expression levels of lncRNAs (LINC01705 and LOC102723403) in 2 lncRNA in exosomes were not significantly different between the two groups (p > 0.05, FIG. 5). In particular, the results showed that lncRNAs LOC105377989 and LOC107986997 in exosomes were most significantly upregulated in the atrial fibrillation group compared to the non-atrial fibrillation group (p < 0.01). Therefore, the lncRNAs LOC105377989 and LOC107986997 in exosomes that were differentially expressed with the greatest statistical significance were selected for the second validation phase.

### [Example 5]

### Second validation of lncRNAs in serum exosomes

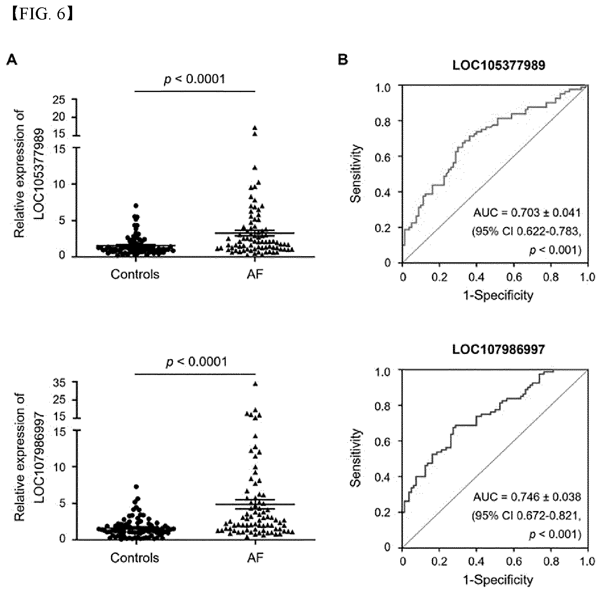

In order to reconfirm the expression levels of the 2 lncRNAs in exosomes selected through the first validation phase, a second validation was performed by targeting 80 patients of the non-atrial fibrillation group and 80 patients of the atrial fibrillation group. As a result, lncRNA LOC105377989 (fold change = 1.72, p < 0.0001) and lncRNA LOC107986997 (fold change = 3.24, p < 0.0001) were significantly upregulated in the atrial fibrillation group compared to the non-atrial fibrillation group (FIG. 6A). Subsequently, ROC curve analysis was used to determine whether the 2 lncRNAs in exosomes could function as potential biomarkers for atrial fibrillation.

As a result, the lncRNAs LOC105377989 and LOC107986997 in exosomes showed AUC = 0.703 ± 0.041 (95% confidence interval (CI) 0.622-0.783, p < 0.001) and AUC = 0.746 ± 0.038 (95% CI 0.672-0.821, p < 0.001), respectively (FIG. 6B). In particular, the lncRNA LOC107986997 in exosomes showed a more significant relationship with atrial fibrillation than lncRNALOC105377989.

### [Example 6]

### Prediction of target gene of IncRNA in serum exosomes

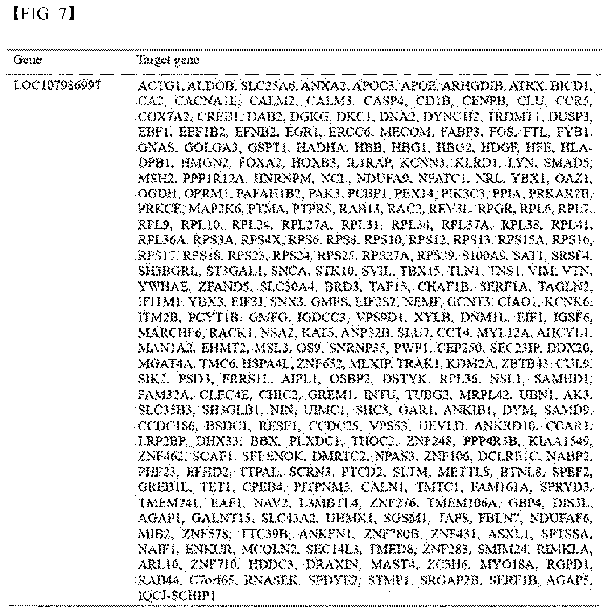

In order to investigate the function of the lncRNA LOC107986997 in exosomes associated with atrial fibrillation, Pearson's correlation coefficient (Irl > 0.8 and p < 0.05) was used to predict a potential target gene for lncRNA LOC107986997 (FIG. 7).

### [Example 7]

### Analysis of target gene function of IncRNA in serum exosomes

Afterwards, GO enrichment analysis was performed by using the PANTHER classification system to determine the function of a target gene. As a result, the target gene of lncRNA LOC107986997 in exosomes was significant in 34 GO terms, and particularly, it was confirmed that it was involved the negative regulation of calcium ion export across plasma membrane, cellular nitrogen compound metabolic and biosynthetic process, and the positive regulation of receptor-mediated endocytosis which were associated with the pathophysiological mechanism of AF (Table 1).

**[Table 1]**

| GO ID | GO term | Count | *P* value | FDR |
|---|---|---|---|---|
| GO:0002181 | cytoplasmic translation | 32 | 4.53E-28 | 7.12E-24 |
| GO:0006412 | Translation | 39 | 3.65E-21 | 2.87E-17 |
| GO:0043043 | peptide biosynthetic process | 39 | 2.42E-20 | 1.27E-16 |
| GO:0043604 | amide biosynthetic process | 42 | 3.61E-19 | 1.42E-15 |
| GO:0034645 | cellular macromolecule biosynthetic process | 66 | 1.23E-17 | 3.22E-14 |
| GO:1901566 | organonitrogen compound biosynthetic process | 63 | 1.05E-17 | 3.31E-14 |
| GO:0009059 | macromolecule biosynthetic process | 66 | 2.75E-17 | 6.17E-14 |
| GO:0044271 | cellular nitrogen compound biosynthetic process | 62 | 1.7E-16 | 3.33E-13 |
| GO:0006518 | peptide metabolic process | 39 | 2.48E-16 | 4.33E-13 |
| GO:0009058 | biosynthetic process | 84 | 4.63E-14 | 6.61E-11 |
| GO:0044249 | cellular biosynthetic process | 81 | 4.56E-14 | 7.17E-11 |
| GO:1901576 | organic substance biosynthetic process | 82 | 5.65E-14 | 7.4E-11 |
| GO:0043603 | cellular amide metabolic process | 43 | 1.89E-13 | 2.28E-10 |
| GO:0010467 | gene expression | 71 | 2.46E-12 | 2.76E-09 |
| GO:0034641 | cellular nitrogen compound metabolic process | 93 | 2.11E-11 | 2.21E-08 |
| GO:0044260 | cellular macromolecule metabolic process | 108 | 1.14E-08 | 0.0000112 |
| GO:0044267 | cellular protein metabolic process | 86 | 1.39E-08 | 0.0000128 |
| GO:0044237 | cellular metabolic process | 149 | 3.66E-08 | 0.000032 |
| GO:0043170 | macromolecule metabolic process | 128 | 4. 11E-08 | 0.000034 |
| GO:0019538 | protein metabolic process | 94 | 8.43E-08 | 0.0000663 |
| GO:0006807 | nitrogen compound metabolic process | 137 | 9.74E-08 | 0.0000729 |
| GO:0044238 | primary metabolic process | 143 | 3.46E-07 | 0.000248 |
| GO:0008152 | metabolic process | 158 | 4.24E-07 | 0.00029 |
| GO:1901564 | organonitrogen compound metabolic process | 108 | 6.17E-07 | 0.000404 |
| GO:0071704 | organic substance metabolic process | 149 | 1.43E-06 | 0.000832 |
| GO:0045807 | positive regulation of endocytosis | 10 | 5.68E-06 | 0.00319 |
| GO:0022613 | ribonucleoprotein complex biogenesis | 19 | 0.0000234 | 0.0127 |
| GO:0048260 | positive regulation of receptor-mediated endocytosis | 7 | 0.0000287 | 0.0151 |
| GO:0042254 | ribosome biogenesis | 15 | 0.0000411 | 0.0208 |
| GO:0070527 | platelet aggregation | 6 | 0.0000765 | 0.0375 |
| GO:0030100 | regulation of endocytosis | 12 | 0.0000906 | 0.0432 |
| GO:1905913 | negative regulation of calcium ion export across plasma membrane | 3 | 0.0000973 | 0.0437 |
| GO:0009987 | cellular process | 248 | 0.0000956 | 0.0442 |
| GO:0006996 | organelle organization | 75 | 0.00011 | 0.048 |

### [Industrial Applicability]

The lncRNA according to the present invention is a biomarker for diagnosing atrial fibrillation, and since it can improve patient convenience and effectively diagnose or prevent atrial fibrillation in a minimally invasive manner, it has industrial applicability.

## Claims

1. A biomarker composition for diagnosing atrial fibrillation, comprising at least one lncRNA selected from the group consisting of LOC105377989, LOC105375434, LOC107986997 and LOC101927073 as an active ingredient.

2. A biomarker composition for diagnosing atrial fibrillation, comprising an agent for measuring the expression level of at least one lncRNA selected from the group consisting of LOC105377989, LOC105375434, LOC107986997 and LOC 101927073 .

3. The biomarker composition of claim 2, wherein the agent for measuring the expression level of the lncRNA is a primer or probe that specifically binds to the lncRNA.

4. A kit for diagnosing atrial fibrillation, comprising the composition of claim 2 or 3.

5. A method for providing information that is required for diagnosing atrial fibrillation, comprising the step of measuring the expression level of at least one lncRNA selected from the group consisting of LOC105377989, LOC105375434, LOC107986997 and LOC101927073 in a sample isolated from a subject.

6. The method of claim 5, wherein the sample is blood or blood-derived serum exosomes.

7. The method of claim 5, wherein the method further comprises the step of determining atrial fibrillation when the result of comparing the measured expression level of lncRNA with the expression level of lncRNA of a normal control sample corresponds to any one of a) to d) below:
a) upregulation of LOC105377989;
b) upregulation of LOC105375434;
c) upregulation of LOC107986997; and
d) downregulation of LOC101927073.

8. A method for screening a therapeutic agent for atrial fibrillation, comprising the step of treating an atrial fibrillation therapeutic agent candidate material to exosomes isolated from mammals with atrial fibrillation except humans or atrial fibrillation patients, and then measuring the expression level of at least one lncRNA selected from the group consisting of LOC105377989, LOC105375434, LOC107986997 and LOC101927073.

9. The method of claim 8, further comprising the step of determining an atrial fibrillation therapeutic agent, when the expression level of lncRNA after treatment with the atrial fibrillation therapeutic agent candidate material compared to before treatment with the atrial fibrillation therapeutic agent candidate material corresponds to any one of a) to d) below:
a) downregulation of LOC105377989;
b) downregulation of LOC105375434;
c) downregulation of LOC107986997; and
d) upregulation of LOC101927073.
